# EUROPÄISCHE PATENTSCHRIFT

(11) **EP 2 648 596 B1**
(45) Veröffentlichungstag und Bekanntmachung des Hinweises auf die Patenterteilung: **08.03.2017**
(21) Anmeldenummer: 11772889.9
(22) Anmeldetag: 15.10.2011
(51) Int. Cl.: A61B 1/00, G02B 23/24, A61B 1/002, A61B 1/018, A61B 1/04, A61B 1/12

(54) **ENDOSKOP**
ENDOSCOPE
ENDOSCOPE

(30) Priorität: 09.12.2010 DE 102010053882
(43) Veröffentlichungstag der Anmeldung: 16.10.2013
(73) Patentinhaber: Schölly Fiberoptic GmbH, 79211 Denzlingen (DE)
(72) Erfinder: HOFER, Axel, 79346 Endingen (DE)
(74) Vertreter: Börjes-Pestalozza, Henrich
(86) Internationale Anmeldenummer: PCT/EP2011/005181
(87) Internationale Veröffentlichungsnummer: WO 2012/076071

(56) Entgegenhaltungen:
- EP-A1- 1 371 321
- WO-A1-96/39916
- US-A- 4 651 201
- US-A- 5 381 784
- US-A- 6 142 932
- US-A1- 2004 249 246
- US-A1- 2010 261 961

## Beschreibung

Die Erfindung betrifft ein Endoskop mit einer zwischen einem proximalen Ende und einem distalen Ende angeordneten optischen Anordnung, wobei am proximalen Ende eine Strahlaufspaltvorrichtung vorgesehen ist und am distalen Ende zwei zur Erfassung eines stereoskopischen Bildes nebeneinander angeordnete Bilderfassungsbereiche ausgebildet sind, wobei die Strahlaufspaltvorrichtung einen Strahlengang der optischen Anordnung derart auf zwei Bildauswertemittel aufteilt, dass jeder: der zwei Bilderfassungsbereiche jeweils einem der zwei Bildauswertemittel zugeordnet ist.

Derartige Systeme sind bekannt und dienen dazu, mit einem Monoendoskop stereoskopisches Sehen zu ermöglichen. Die Grundidee dieser stereoskopisch genutzten Monoendoskope ist, dass Randbereiche der optischen Strahlengänge der optischen Anordnung ausgenutzt werden, um die für das stereoskopische Sehen erforderlichen zwei Strahlengänge zu bilden.

Bei den bisher bekannten Endoskopen ist es üblich, die optische Anordnung mit einem Lichtleiterbündel zu umgeben, welches im Querschnitt kreisringförmig ausgebildet ist und die optische Anordnung in der Mitte des Kreisrings aufnimmt. Mit diesem Lichtleiterbündel ist eine Ausleuchtung der betrachteten Szene möglich, jedoch wird hierdurch der Querschnitt des Endoskops gegenüber der optischen Anordnung vergrößert, was insbesondere bei minimal invasiven Eingriffen äußerst unerwünscht ist.

Aus US 6 142 932 A ist der Aufbau eines Vorderendes eines stereoskopischen Endoskops mit einer langgestreckten Linse bekannt, bei welchem zwei Bildaufnahmensensoren im distalen Ende des Endoskops angeordnet sind.

Aus US 5 381 784 A ist ein stereoskopisches Endoskop bekannt, bei welchem eine Endkappe an dem distalen Ende eines zylindrischen Rohres angelenkt ist, wobei eine erste Objektivoptik in dem Rohr und eine zweite Objektivoptik in der abklappbaren Kappe angeordnet sind.

Aus US 2004/0249246 A1 sind ein System, eine Vorrichtung und ein Verfahren zur Betrachtung eines visuell verdeckten Bereichs eines Hohlraums bekannt, wobei die optischen Kanäle zum stereoskopischen Sehen durch voneinander getrennt geführte Linsensysteme gebildet sind.

Der Erfindung liegt die: Aufgabe zugrunde, die Gebrauchseigenschaften eines stereoskopisch genutzten Endoskops zu verbessern.

Zur Lösung dieser Aufgabe sind die Merkmale des Anspruchs 1 vorgesehen. Insbesondere ist somit bei einem Endoskop der eingangs genannten Art vorgesehen, dass zumindest in einem distalen Abschnitt die optische Anordnung einen unrunden Querschnitt quer zu einer optischen Achse der optischen Anordnung aufweist und dass zumindest in dem distalen Abschnitt innerhalb eines den Querschnitt umbeschriebenen Umkreises ein längs oder parallel der optischen Achse verlaufender Funktionskanal ausgebildet ist. Die Erfindung macht sich somit die Erkenntnis zu Nutze, dass wegen der versetzt nebeneinander angeordneten Bilderfassungsbereiche für das stereoskopische Sehen die optische Anordnung in ihrem Querschnitt in einer Richtung, nämlich der Richtung der Verbindungslinie der Zentren der Bilderfassungsbereiche, einen größeren Platzbedarf hat, als in der senkrecht zu dieser Richtung in der Querschnittsebene liegende Richtung. Hierdurch wird in einer Richtung eine maximale Abmessung und in einer senkrechten Richtung dazu eine minimal erforderliche Abmessung vorgegeben, welche die Bilderfassungsbereiche gerade noch aufnimmt. Betrachtet man einen der optischen Anordnung im Querschnitt umbeschriebenen Umkreis, welcher somit einen Durchmesser aufweist, der gleich der erwähnten maximalen Abmessung ist, so ergeben sich durch die kleinere minimale erforderliche Abmessung quer zu dieser Richtung Bereiche innerhalb des Umkreises, die für das stereoskopische Sehen nicht benötigt werden und die daher für andere Zwecke genutzt werden können. Die Erfindung schlägt vor, in diesen Bereichen einen Funktionskanal auszubilden. Somit bietet die Erfindung den Vorteil, dass ohne eine Verkleinerung oder Beschneidung der Bilderfassungsbereiche, welche eine Verschlechterung der erfassten Bildqualität bedeuten würde, der Gesamtquerschnitt des Endoskops und insbesondere die maximale Querabmessung verringert werden können. Dies verbessert die Gebrauchseigenschaften des Endoskops erheb lich. Unter einem umbeschriebenen Umkreis wird hierbei derjenige Kreis verstanden, der alle Punkte des umbeschriebenen Querschnitts einschließt bzw. berührt und der dabei einen minimalen Durchmesser aufweist.

Der unrunde Querschnitt kann beispielsweise durch eine Abflachung bzw. Abplattung eines kreisrunden Querschnitts, beispielsweise durch einen ebenen Anschliff, gebildet sein.

Der Funktionskanal kann hierbei nur teilweise innerhalb des Umkreises angeordnet sein und über diesen hinausragen. Bevorzugt ist der Funktionskanal vollständig innerhalb des Umkreises ausgebildet.

Der distale Abschnitt kann wenigstens das distale Ende umfassen und erstreckt sich vorzugsweise über die Distanz zwischen dem proximalen Ende und dem distalen Ende. Der distale Abschnitt erstreckt sich vorzugsweise zumindest über denjenigen Abschnitt des Endoskops, der zur Einführung in den zu untersuchenden Hohlraum bestimmt ist.

Eine besonders günstige Ausnutzung der optischen Element wird erreicht, wenn der unrunde Querschnitt durch eine Abmessung und/oder einen Abstand der Bilderfassungsbereiche vorgegeben ist. Hierbei kann die Abmessung durch den Durchmesser jedes Bilderfassungsbereiches gegeben sein. Der Abstand kann durch den geometrischen Abstand der Zentren der Bilderfassungsbereiche gegeben sein. Die Bilderfassungsbereiche können sich demnach überlappen oder disjunkt zueinander angeordnet sein. Besonders günstig ist es, wenn der Querschnitt der optischen Anordnung so gewählt ist, dass die Bilderfassungsbereiche durch die Begrenzung des Querschnitts gerade nicht beschnitten werden.

Bei einer Ausgestaltung der Erfindung kann vorgesehen sein, dass die optische Anordnung in einem Aufnahmekanal angeordnet ist. Von Vorteil ist dabei, dass eine definierte Positionierung und Ausrichtung der optischen Elemente der optischen Anordnung möglich ist.

Um die optische Anordnung im Gebrauch nicht zu verunreinigen und/oder um das Endoskop einfach reinigen zu können, kann vorgesehen sein, dass der Aufnahmekanal gegen den Funktionskanal dicht abgeschlossen ist.

Es kann vorgesehen sein, dass der Funktionskanal zwischen seinem distalen Ende und seinem proximalen Ende nach außen dicht abgeschlossen ausgebildet ist. Auf diese Weise kann ein seitliches Eindringen von Flüssigkeiten und dergleichen vermieden werden, was die Gebrauchseigenschaften des Endoskops nochmals steigert.

Beispielsweise kann vorgesehen sein, dass der unrunde Querschnitt gegenüber einer Kreisform, insbesondere gegenüber dem Umkreis, an zwei einander gegenüberliegenden Seiten eine Abplattung aufweist. Es sind somit ovale oder elliptische Formeln bildbar. Eine besonders einfach zu fertigende Querschnittsform kann vorsehen, dass der Querschnitt aus einer Kreisform gebildet ist, wobei die Abplattungen durch Wegnahme von Kreissegmenten, auch Kreisabschnitte genannt, gebildet ist. Vorzugsweise sind hierbei die den Kreissegmenten zugeordneten Sehnen parallel zueinander ausgebildet. Von Vorteil ist dabei, dass sich die Wegnahme von Kreissegmenten aus einem kreisförmigen Querschnitt durch ebenes Anschleifen einfach fertigen lässt.

Für eine möglichst optimale Ausnutzung des von der Anordnung der Bilderfassungsbereiche geforderten Querschnitts kann vorgesehen sein, dass zumindest in dem distalen Abschnitt auf zwei im Querschnitt der optischen Anordnung einander gegenüberliegenden Seiten jeweils ein längs der optischen Achse verlaufender Funktionskanal ausgebildet ist.

Um einfache Verhältnisse der Strahlführung für das stereoskopische Sehen zu ermöglichen, kann vorgesehen sein, dass die optische Anordnung und der wenigstens eine Funktionskanal bzw. die Funktionskanäle in einem Rohr angeordnet sind. Dieses Rohr kann als starres Rohr ausgebildet sein. Von Vorteil ist dabei, dass die optischen Elemente der optischen Anordnung in ihre Relativposition zueinander fixiert sind. Dies vereinfacht die Verhältnisse für das stereoskopische Sehen.

Das Rohr kann in seinem Rohrquerschnitt Platz für einen zusätzlichen Funktionskanal bieten, welcher die optische Anordnung radial umgibt und einschließt.

Um ein Endoskop mit möglichst minimalem Gesamtquerschnitt zu bilden, kann vorgesehen sein, dass ein Rohrquerschnitt des Rohres quer zur optischen Achse durch eine Abmessung des Querschnitts der optischen Anordnung bestimmt ist. Es kann vorgesehen sein, dass das Rohr der optischen Anordnung umbeschrieben ist. Von Vorteil ist dabei, dass der mindestens notwendige Querschnitt optimal ausgenutzt werden kann und dass kein Platz verschenkt wird.

Für eine vielseitige Einsatzmöglichkeit kann vorgesehen sein, dass in dem Funktionskanal ein Lichtleiterbündel angeordnet ist. Es hat sich als günstig erwiesen, wenn dieses Lichtleiterbündel herausnehmbar angeordnet ist, um beispielsweise während einer Reinigung herausgenommen werden zu können oder um gegen ein anderes Arbeitsmittel ausgetauscht werden zu können.

Sofern an dem Endoskop mehrere Funktionskanäle erfindungsgemäß ausgebildet sind, können einige oder alle dieser Funktionskanäle mit Lichtleiterbündeln bestückt sein.

Es kann auch vorgesehen sein, dass der oder ein Funktionskanal an seinem proximalen Ende einen Anschluss für eine Saug- und/oder Spülvorrichtung aufweist. Von Vorteil ist dabei, dass die Einsatzmöglichkeiten des erfindungsgemäßen Endoskops noch einmal vergrößert werden.

Der erfindungsgemäße Funktionskanal bzw. die erfindungsgemäßen Funktionskanäle ist/sind auch anderweitig, beispielsweise für Instrumente und/oder Zusatzgeräte, nutzbar.

Die Erfindung wird nun anhand von einem Ausführungsbeispiel näher beschrieben, ist aber nicht auf dieses Ausführungsbeispiel beschränkt. Weitere Ausführungsbeispiele ergeben sich durch Kombination einzelner oder mehrerer Merkmale der Patentansprüche untereinander und/oder mit einzelnen oder mehreren Merkmalen des Ausführungsbeispiels.

Es zeigt:
- Fig. 1: in einer dreidimensionalen Schrägansicht die Prinzipskizze eines erfindungsgemäßen Endoskops,
- Fig. 2: die Anordnung gemäß Figur 1 in einer Draufsicht mit typischen Strahlverläufen des stereoskopischen Sehens,
- Fig. 3: eine Ansicht entlang einer optischen Achse auf das distale Ende des Endoskops gemäß Figur 1 und
- Fig. 4: eine Ansicht auf das distale Ende entsprechend Figur 3 mit befüllten Funktionskanälen.

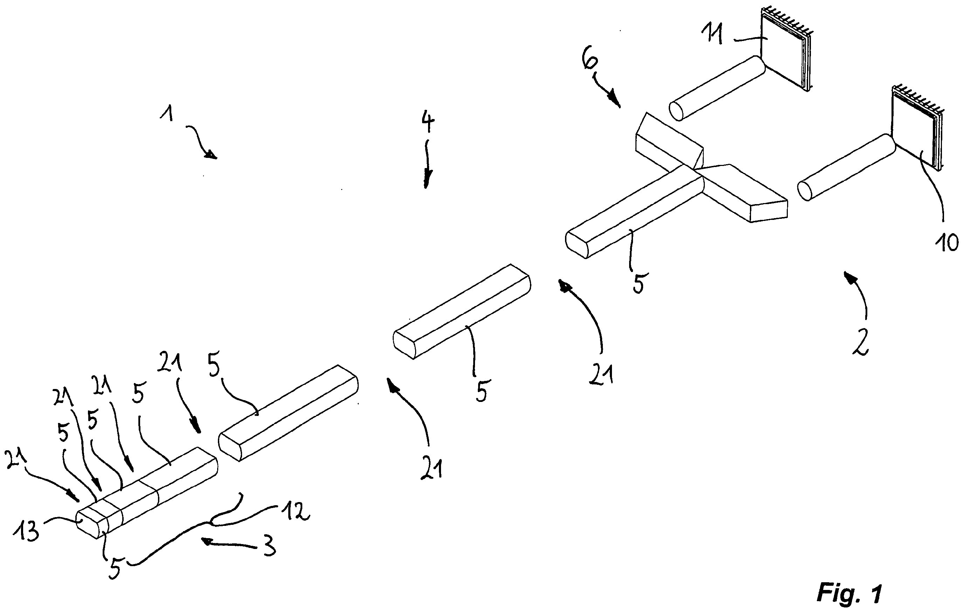

Figur 1 zeigt in einer dreidimensionalen Schrägansicht eine Prinzipskizze eines im Ganzen mit 1 bezeichneten erfindungsgemäßen Endoskops. In dieser Prinzipskizze sind an sich bekannte Elemente des Endoskops 1, die zur Erläuterung der Erfindung nicht unbedingt notwendig sind, zur Vereinfachung weggelassen.

Das Endoskop 1 hat ein proximales, also dem Benutzer in Gebrauchsstellung zugewandtes Ende 2 und ein distales, also in Gebrauchsstellung vom Benutzer abgewandtes Ende 3.

Zwischen dem proximalen Ende 2 und dem distalen Ende 3 ist eine optische Anordnung 4 ausgebildet. Die optische Anordnung 4 umfasst hintereinander angeordnete optische Elemente 5, beispielsweise Stablinsen oder Linsen.

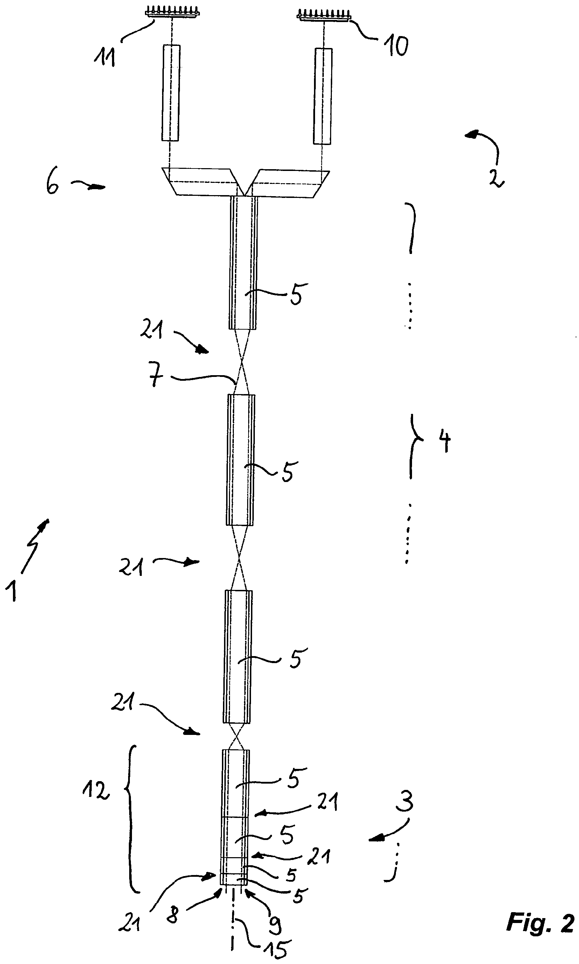

Am proximalen Ende 2 ist eine Strahlaufspaltvorrichtung 6 vorgesehen, welche in an sich bekannter Weise einen in Figur 2 schematisch angedeuteten Strahlengang 7 aufteilt.

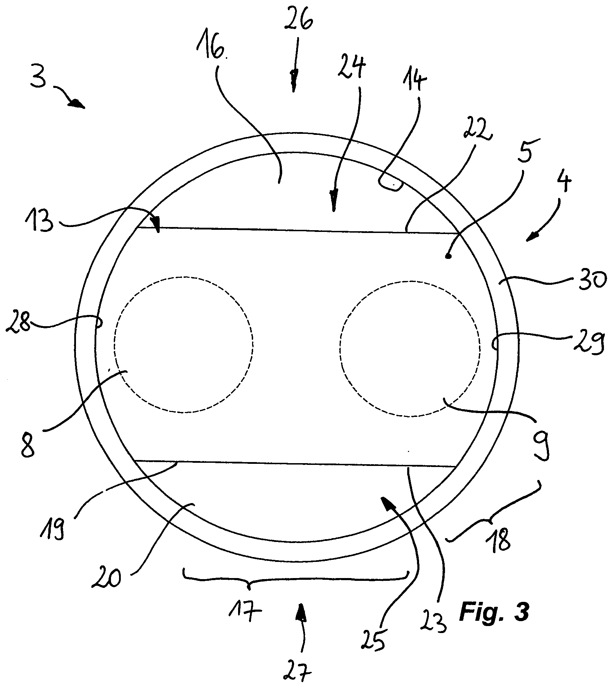

Die Strahlaufspaltvorrichtung 6 sorgt daher dafür, dass die am distalen Ende 3 zum stereoskopischen Sehen versetzt nebeneinander angeordneten Bilderfassungsbereiche 8, 9 (vgl. Fig.3) durch die Bildauswertemittel 10, 11 derart erfasst werden, dass der Bilderfassungsbereich 8 auf das Bildauswertemittel 10 projektiert wird, während der Bilderfassungsbereich 9 auf das Bildauswertemittel 11 projektiert wird.

Somit können die mit den Bilderfassungsbereichen 8, 9 erfassten Bilder durch die Bildauswertemittel 10, 11 getrennt ausgewertet und für ein stereoskopisches Sehen bereitgestellt werden.

Im Ausführungsbeispiel gemäß den Figuren sind die Bildauswertemittel 10, 11 durch Bildaufnahmechips realisiert. Die Bildauswertemittel können jedoch bei weiteren Ausführungsbeispielen auch durch Okulare zur direkten Beobachtung gegeben sein.

Zumindest in einem distalen Abschnitt 12 weist die optische Anordnung 4 einen unrunden Querschnitt 13 auf, welcher von den optischen Elementen 5 ausgefüllt wird.

Im gezeigten Ausführungsbeispiel weist die optische Anordnung 4 über ihre gesamte Erstreckung zwischen dem proximalen Ende 2 und dem distalen Ende 3 diesen unrunden Querschnitt 13 auf. Wie in Figur 3 zu erkennen ist, wird durch den unrunden Querschnitt 13 gegenüber einem die optische Anordnung 4 unbeschreibenden Umkreis 14 ein Freiraum gebildet, welcher einen längs oder parallel einer optischen Achse 15 verlaufenden Funktionskanal 16 bildet.

Der unrunde Querschnitt 13 ist hierbei gemäß Fig.3 so durch eine Abmessung 17, nämlich den geometrischen Abstand der Zentren der Bilderfassungsbereiche 8, 9 voneinander, und durch eine Abmessung 18, nämlich den geometrischen Durchmesser jedes der Bilderfassungsbereiche 8, 9, so vorgegeben und bemessen, dass eine störungsfreie Projektion der Bilderfassungsbereiche 8, 9 auf das jeweilige Bildauswertemittel 10, 11 ermöglicht ist, ohne die Bilderfassungsbereiche 8, 9 zu beschneiden.

Die optische Anordnung 4 ist in einem Aufnahmekanal 19 angeordnet, welcher gegen den Funktionskanal 16 und einen zweiten Funktionskanal 20 dicht abgeschlossen ist.

Insbesondere sind somit die in Figur 1 ersichtlichen Zwischenräume 21 zwischen den optischen Elementen 5 der optischen Anordnung 4 durch den Aufnahmekanal 19 dicht verschlossen.

Ebenso ist der Funktionskanal 16 zwischen dem distalen Ende 3 und dem proximalen Ende 2 nach außen dicht abgeschlossen ausgebildet.

Der unrunde Querschnitt 13 ist bei dem in Figur 3 gezeigten Ausführungsbeispiel dadurch gebildet, dass das ursprünglich mit kreisrundem Querschnitt gefertigte optische Element 5 an zwei gegenüberliegenden Seiten 26, 27 eben angeschliffen wurde. Hierdurch sind somit gegenüber dem kreisrunden Querschnitt, welcher durch den Umkreis 14 gegeben ist, zwei Kreissegmente weggenommen. Hierdurch beschreiben die im Schleifvorgang herausgebildeten geometrischen Sehnen 22, 23 des unrunden Querschnitts 13 Abplattungen 24, 25.

Zwischen den ebenen Abplattungen 24, 25 beschreibt der unrunde Querschnitt 13 jeweils eine kreisbogenförmige Kontur 28, 29.

Durch die Abplattungen 24, 25 ist gegenüber dem ursprünglichen Querschnitt des unbearbeiteten optischen Elements 5, welcher durch den Umkreis 14 beschrieben ist, auf jeder Seite 26, 27 ein Freiraum für einen Funktionskanal 16, 20 gebildet.

Die optische Anordnung 4 und die Funktionskanäle 16, 20 sind in einem starren Rohr 30 angeordnet, welches sich zwischen dem proximalen Ende 2 und dem distalen Ende 3 erstreckt.

Hierbei ist der Rohrquerschnitt des Rohres 30 quer zur optischen Achse 15 durch die Abmessung des Querschnitts 13 der optischen Anordnung 4 bestimmt. Insbesondere ist dieses Rohr 30 der optischen Anordnung 4 umbeschrieben. Die Funktionskanäle 16, 20 bedingen somit keinen vergrößerten Rohrquerschnitt des Rohres 30.

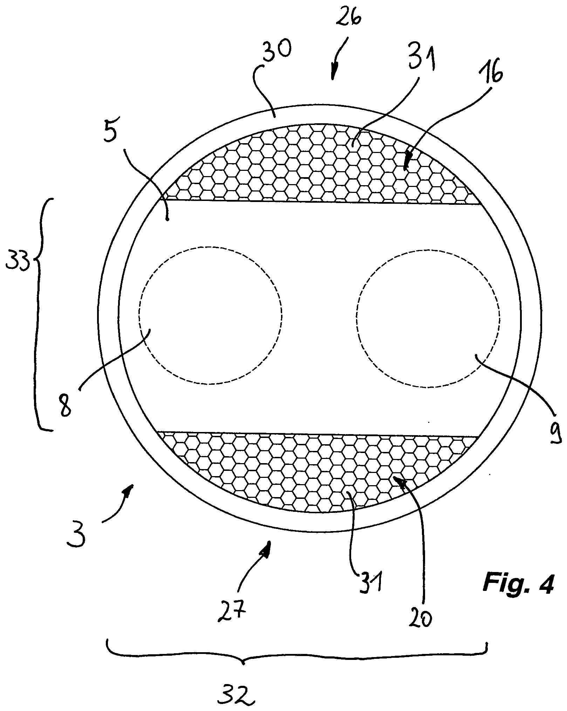

Figur 4 zeigt eine mögliche Belegung der Funktionskanäle 16, 20 mit jeweils einem Lichtleiterbündel 31.

Die Lichtleiterbündel 31 sind in den Funktionskanälen 16, 20 herausnehmbar angeordnet und können einzeln oder gemeinsam für eine Reinigung oder für eine andere Funktionsbelegung herausgezogen werden.

Beispielsweise kann einer der Funktionskanäle 16, 20 zum Absaugen oder zum Spülen am distalen Ende 3 verwendet werden, wobei in diesem Fall am proximalen Ende 2 des entsprechenden Funktionskanals 16, 20 eine an sich bekannte und hier nicht weiter dargestellte Saug- und/oder Spülvorrichtung angeschlossen ist.

Bei dem Endoskop 1 zum stereoskopischen Sehen ist vorgesehen, die zur Erfassung des stereoskopischen Bildes vorhandene optische Anordnung 4 mit einem unrunden Querschnitt 13 auszubilden und in wenigstens einem gegenüber der kreisrunden Querschnittsform 14 abgeflachten Bereich einen zusätzlichen Funktionskanal 16, 20 auszubilden.

## Patentansprüche

1. Endoskop (1) mit einer zwischen einem proximalen Ende (2)und einem distalen Ende (3) angeordneten optischen Anordnung (4), wobei am proximalen Ende (2) eine Strahlaufspaltvorrichtung (6) vorgesehen ist und am distalen Ende (3) zwei zur Erfassung eines stereoskopischen Bildes nebeneinander angeordnete Bilderfassungsbereiche (8, 9) ausgebildet sind, wobei die Strahlaufspaltvorrichtung (6) einen Strahlengang (7) der optischen Anordnung (4) derart auf zwei Bildauswertemittel (10, 11) aufteilt, dass jeder der zwei Bilderfassungsbereiche (8, 9) jeweils einem der zwei Bildauswertemittel (10, 11) zugeordnet ist, was dadurch erreicht wird, dass Randbereiche des optischen Strahlengangs (7) der optischen Anordnung (4) ausgenutzt werden, um die für das stereoskopische Sehen erforderlichen zwei Strahlengänge zu bilden, und dass weiterhin zumindest in einem distalen Abschnitt (12) die optische Anordnung (4) einen unrunden Querschnitt (13) quer zu einer optischen Achse (15) der optischen Anordnung (4) aufweist und dass zumindest in dem distalen Abschnitt (12) innerhalb eines dem Querschnitt (13) umbeschriebenen Umkreises (14) ein längs öder parallel der optischen Achse (15) verlaufender Funktionskanal (16, 20) ausgebildet ist.

2. Endoskop (1) nach Anspruch 1, **dadurch gekennzeichnet, dass** der unrunde Querschnitt (13) durch eine Abmessung (18) und/oder einen Abstand (17) der Bilderfassungsbereiche (8, 9) vorgegeben ist und/oder dass der unrunde Querschnitt (13) durch wenigstens eine Abplattung (24, 25) in Bezug auf den Umkreis (14) gebildet ist.

3. Endoskop (1) nach Anspruch 1 oder 2, **dadurch gekennzeichnet, dass** die optische Anordnung (4) in einem Aufnahmekanal (19) angeordnet ist, insbesondere wobei der Aufnahmekanal (19) gegen den Funktionskanal (16, 20) dicht abgeschlossen ist.

4. Endoskop (1) nach einem der Ansprüche 1 bis 3, **dadurch gekennzeichnet, dass** der Funktionskanal (16, 20) zwischen seinem distalen Ende (2) und seinem proximalen Ende (3) nach außen dicht abgeschlossen ausgebildet ist.

5. Endoskop (1) nach einem der Ansprüche 1 bis 4, **dadurch gekennzeichnet, dass** der unrunde Querschnitt (13) gegenüber einer Kreisform, insbesondere gegenüber dem Umkreis (14), an zwei einander gegenüberliegenden Seiten (26, 27) eine Abplattung (24, 25) aufweist.

6. Endoskop (1) nach einem der Ansprüche 1 bis 5, **dadurch gekennzeichnet, dass** zumindest in dem distalen Abschnitt (12) auf zwei im Querschnitt (13) der optischen Anordnung (4) einander gegenüberliegenden Seiten (26, 27) jeweils ein längs oder parallel der optischen Achse (15) verlaufender Funktionskanal (16, 20) ausgebildet ist.

7. Endoskop (1) nach einem der Ansprüche 1 bis 6, **dadurch gekennzeichnet, dass** die optische Anordnung (4) und der Funktionskanal/die Funktionskanäle (16, 20) in einem vorzugsweise starren Rohr (30) angeordnet sind.

8. Endoskop (1) nach einem der Ansprüche 1 bis 7, **dadurch gekennzeichnet, dass** ein Rohrquerschnitt des Rohres (30) quer zur optischen Achse (15) durch eine Abmessung (32), insbesondere eine maximale Abmessung (32), des Querschnitts (13) der optischen Anordnung (4) bestimmt ist und/oder dass das Rohr (30) der optischen Anordnung (4) umbeschrieben ist.

9. Endoskop (1) nach einem der Ansprüche 1 bis 8, **dadurch gekennzeichnet, dass** in dem oder einem Funktionskanal (16, 20) ein Lichtleiterbündel (31) vorzugsweise herausnehmbar angeordnet ist.

10. Endoskop (1) nach einem der Ansprüche 1 bis 9, **dadurch gekennzeichnet, dass** der oder ein Funktionskanal (16, 20) an seinem proximalen Ende (2) einen Anschluss für eine Saug- und/oder Spülvorrichtung aufweist.

## Claims

1. Endoscope (1) comprising an optical arrangement (4) arranged between a proximal end (2) and a distal end (3), wherein the proximal end (2) has a beam splitting apparatus (6) provided thereon and the distal end (3) has two image recording regions (8, 9) formed thereon which are arranged next to one another for the purpose of recording a stereoscopic image, wherein the beam splitting apparatus (6) divides a beam path (7) of the optical arrangement (4) into two image evaluation means (10, 11) such that each of the two image recording regions (8, 9) is allocated in each case to one of the two image evaluation means (10, 11), which is achieved in that edge regions of the optical beam path (7) of the optical arrangement (4) are utilised in order to form the two beam paths required for stereoscopic vision, and that furthermore at least in a distal portion (12) the optical arrangement (4) has a non-circular cross-section (13) transverse to an optical axis (15) of the optical arrangement (4) and that a functional channel (16, 20) which extends longitudinally to or in parallel with the optical axis (15) is formed at least in the distal portion (12) within a circle (14) encircling the cross-section (13).

2. Endoscope (1) as claimed in claim 1, **characterised in that** the non-circular cross-section (13) is specified by a dimension (18) and/or a spaced interval (17) of the image recording regions (8, 9) and/or that the non-circular cross-section (13) is formed by at least one flattening (24, 25) in relation to the circle (14).

3. Endoscope (1) as claimed in claim 1 or 2, **characterised in that** the optical arrangement (4) is arranged in a receiving channel (19), in particular wherein the receiving channel (19) is closed off in a sealing manner with respect to the functional channel (16, 20).

4. Endoscope (1) as claimed in any one of claims 1 to 3, **characterised in that** the functional channel (16, 20) is formed so as to be closed off outwards in a sealing manner between its distal end (2) and its proximal end (3).

5. Endoscope (1) as claimed in any one of claims 1 to 4, **characterised in that** the non-circular cross-section (13) has a flattening (24, 25) with respect to a circular shape, in particular with respect to the circle (14), on two mutually opposite sides (26, 27).

6. Endoscope (1) as claimed in any one of claims 1 to 5, **characterised in that** a functional channel (16, 20) which extends longitudinally to or in parallel with the optical axis (15) is formed in each case at least in the distal portion (12) on two sides (26, 27) which are mutually opposite in the cross-section (13) of the optical arrangement (4).

7. Endoscope (1) as claimed in any one of claims 1 to 6, **characterised in that** the optical arrangement (4) and the functional channel(s) (16, 20) are arranged in a preferably rigid tube (30).

8. Endoscope (1) as claimed in any one of claims 1 to 7, **characterised in that** a tube cross-section of the tube (30) transverse to the optical axis (15) is determined by a dimension (32), in particular a maximum dimension (32), of the cross-section (13) of the optical arrangement (4) and/or that the tube (30) of the optical arrangement (4) is encircled.

9. Endoscope (1) as claimed in any one of claims 1 to 8, **characterised in that** a light conductor bundle (31) is arranged, preferably in a removable manner, in the or a functional channel (16, 20).

10. Endoscope (1) as claimed in any one of claims 1 to 9, **characterised in that** the or a functional channel (16, 20) has, at its proximal end (2), a connection for a suction and/or flushing apparatus.

## Revendications

1. Endoscope (1) avec un arrangement optique (4) disposé entre une extrémité proximale (2) et une extrémité distale (3), dans lequel il est prévu à l'extrémité proximale (2) un dispositif de dédoublement du rayon (6) et deux zones de détection d'image (8, 9) disposées l'une à côté de l'autre sont formées à l'extrémité distale (3) pour la détection d'une image stéréoscopique, dans lequel le dispositif de dédoublement de rayon (6) divise un trajet de rayon (7) de l'arrangement optique (4) vers deux moyens d'évaluation d'image (10, 11), de telle manière que chacune des deux zones de détection d'image (8, 9) soit respectivement associé à l'un des deux moyens d'évaluation d'image (10, 11), ce qui est obtenu par le fait que des régions de bord du trajet de rayon optique (7) de l'arrangement optique (4) sont utilisées pour former les deux trajets de rayon nécessaires pour la vision stéréoscopique et qu'en outre, l'arrangement optique (4) présente, au moins dans une partie distale (12), une section transversale non ronde (13) transversalement à un axe optique (15) de l'arrangement optique (4) et qu'au moins dans la partie distale (12), un canal de fonction (16, 20) s'étendant le long de ou parallèlement à l'axe optique (15) est formé à l'intérieur d'un cercle (14) circonscrit à la section transversale (13).

2. Endoscope (1) selon la revendication 1, **caractérisé en ce que** la section transversale non ronde (13) est prédéterminée par une dimension (18) et/ou une distance (17) des zones d'évaluation d'image (8, 9) et/ou **en ce que** la section transversale non ronde (13) est formée par au moins un méplat (24, 25) par rapport au cercle circonscrit (14).

3. Endoscope (1) selon la revendication 1 ou 2, **caractérisé en ce que** l'arrangement optique (4) est disposé sur un canal de réception (19), en particulier dans lequel le canal de réception (19) est fermé de façon étanche par rapport au canal de fonction (16, 20).

4. Endoscope (1) selon l'une quelconque des revendications 1 à 3, **caractérisé en ce que** le canal de fonction (16, 20) est fermé de façon étanche vers l'extérieur entre son extrémité distale (2) et son extrémité proximale (3).

5. Endoscope (1) selon l'une quelconque des revendications 1 à 4, **caractérisé en ce que** la section transversale non ronde (13) présente, par rapport à une forme circulaire, en particulier par rapport au cercle circonscrit (14), un méplat (24, 25) sur deux côtés opposés l'un à l'autre (26, 27).

6. Endoscope (1) selon l'une quelconque des revendications 1 à 5, **caractérisé en ce qu'**un canal de fonction (16, 20) s'étendant le long de ou parallèlement à l'axe optique (15) est respectivement formé, au moins dans la partie distale (12), sur deux côtés opposés l'un à l'autre (26, 27) dans la section transversale (13) de l'arrangement optique (4).

7. Endoscope (1) selon l'une quelconque des revendications 1 à 6, **caractérisé en ce que** l'arrangement optique (4) et le canal de fonction/les canaux de fonction (16, 20) est/sont disposé(s) dans un tube de préférence rigide (30).

8. Endoscope (1) selon l'une quelconque des revendications 1 à 7, **caractérisé en ce qu'**une section transversale de tube du tube (30) transversalement à l'axe optique (15) est déterminée par une dimension (32), en particulier une dimension maximale (32), de la section transversale (13) de l'arrangement optique (4) et/ou **en ce que** le tube (30) est circonscrit à l'arrangement optique (4).

9. Endoscope (1) selon l'une quelconque des revendications 1 à 8, **caractérisé en ce qu'**un faisceau de fibres optiques (31), de préférence amovible, est disposé dans le ou dans un canal de fonction (16, 20).

10. Endoscope (1) selon l'une quelconque des revendications 1 à 9, **caractérisé en ce que** le ou un canal de fonction (16, 20) présente à son extrémité proximale (2) un raccord pour un dispositif d'aspiration et/ou de rinçage.
